(19) Europäisches Patentamt
European Patent Office
Office européen des brevets

(11) Veröffentlichungsnummer: **0 144 847**
**A2**

# (12) EUROPÄISCHE PATENTANMELDUNG

(21) Anmeldenummer: **84113929.8**

(22) Anmeldetag: **17.11.84**

(51) Int. Cl.[4]: **C 07 D 491/048**
A 61 K 31/435, C 07 F 7/10
A 61 K 31/50, A 61 K 31/505
A 61 K 31/495, A 61 K 31/47
//(C07D491/048, 307:00, 221:00)

(30) Priorität: **02.12.83 DE 3343658**

(43) Veröffentlichungstag der Anmeldung:
**19.06.85 Patentblatt 85/25**

(84) Benannte Vertragsstaaten:
**AT BE CH DE FR GB IT LI**

(71) Anmelder: **BAYER AG**
**Konzernverwaltung RP Patentabteilung**
**D-5090 Leverkusen 1 Bayerwerk(DE)**

(72) Erfinder: **Görlitzer, Klaus, Prof. Dr.**
**Rothenburgstrasse 25**
**D-1000 Berlin 41(DE)**

(72) Erfinder: **Bartke, Ulrich**
**von Wettsteinstrasse 11**
**D-1000 Berlin 33(DE)**

(72) Erfinder: **Goldmann, Siegfried, Dr.**
**Kuckuckstrasse 41**
**D-5600 Wuppertal 2(DE)**

(72) Erfinder: **Schramm, Matthias, Dr.**
**Paffrather Strasse 38**
**D-5000 Koeln 80(DE)**

(72) Erfinder: **Thomas, Günter, Dr.**
**Claudiusweg 9**
**D-5600 Wuppertal 1(DE)**

(72) Erfinder: **Gross, Rainer, Dr.**
**Platzhoffstrasse 23**
**D-5600 Wuppertal 1(DE)**

## (54) Hydroxy-tetrahydropyridinlactone, Verfahren zu ihrer Herstellung und ihre Verwendung in Arzneimitteln.

(57) Verbindungen der Formel

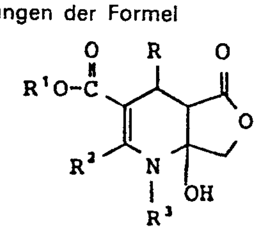

mit den in der Beschreibung genannten Bedeutungen für die Substituenten R, $R_1$, $R_2$ und $R_3$, ein Verfahren zu ihrer Herstellung durch Umsetzung von Verbindungen der Formel

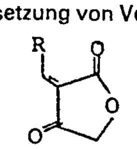

mit Enaminen der Formel

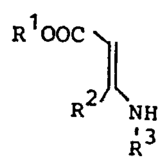

sowie ihre Verwendung bei der Bekämpfung von Erkrankungen.

EP 0 144 847 A2

Croydon Printing Company Ltd

0144847

BAYER AKTIENGESELLSCHAFT 5090 Leverkusen, Bayerwerk

Konzernverwaltung RP
Patentabteilung E/Kü-c

# Hydroxy-tetrahydropyridinlactone, Verfahren zu ihrer Herstellung und ihre Verwendung in Arzneimitteln

Die vorliegende Erfindung betrifft neue 2-Hydroxy-1,2,3,4-tetrahydropyridinlactone, ein Verfahren zu ihrer Herstellung sowie ihre Verwendung in Arzneimitteln, insbesondere in kreislaufbeeinflussenden Arzneimitteln.

Die neuen Verbindungen sind durch folgende Formel (I) gekennzeichnet:

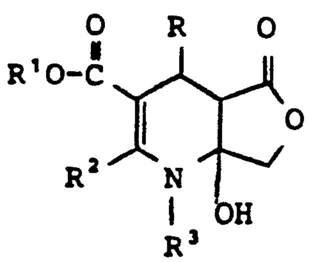

(I)

in welcher

R für einen Phenyl-, Naphthyl-, Thienyl-, Furyl-, Pyrryl-, Pyrazolyl-, Imidazolyl-, Oxazolyl-, Isoxazolyl-, Thiazolyl-, Pyridyl-, Pyridazinyl-,

Pyrimidyl-, Pyrazinyl-, Chinolyl-, Isochinolyl-, Indolyl-, Benzimidazolyl-, Chinazolyl-, Chinoxalyl-, Thionaphthenyl-, Isothionaphthenyl-, Chromonyl-, Thiochromonyl-, Chromenyl-, Thiochromenyl-, Benzoxadiazolyl- oder Benzthiadiazolylrest steht, wobei die genannten Reste gegebenenfalls 1 bis 3 gleiche oder verschiedene Substituenten aus der Gruppe Alkyl- (1 bis 20 Kohlenstoffatome), Alkenyl- (2 bis 20 Kohlenstoffatome), Alkinyl (7 bis 20 C-Atome), Alkoxy (1 bis 20 C-Atome), Fluor, Chlor, Brom, Jod, Trifluormethyl, Monofluoralkoxy- (1 bis 10 C-Atome), Polyfluoralkoxy- (1 bis 10 C-Atome), Hydroxy-, Amino, Monoalkylamino- (1 bis 10 C-Atome), Dialkylamino- (1 bis 10 C-Atome), Nitro, Cyano, Azido, Carboxy, Carbalkoxy($C_1$-$C_{10}$), Carbonamido, Sulfonamido, $SO_m$-Alkyl (m = 0 bis 2, 1 bis 20 C-Atome), Phenyl-, Benzyl-, Benzyloxy- oder Benzylthio- enthalten können, wobei die 4 letztgenannten Substituenten gegebenenfalls 1 bis 3 Reste aus der Gruppe Alkyl (1 bis 5 C-Atome), Alkoxy (1 bis 5 C-Atome), Alkylthio (1 bis 5 C-Atome), Fluor, Chlor, Brom, Jod, Cyano, Nitro, Azido, Hydroxy, Trifluormethyl-, Amino-, Alkylamino- (1 bis 6 C-Atome) oder Dialkylamino- (1 bis 6 C-Atome) enthalten können,

$R^1$ für einen geradkettigen, verzweigten oder cyclischen gesättigten oder ungesättigten Kohlenwasserstoffrest (1 bis 20 C-Atome) steht, der gegebenenfalls durch 1 oder 2 Sauerstoff- oder Schwefelatome in der Kette unterbrochen sein kann und der gegebenenfalls substituiert sein kann durch ein oder mehrere Fluoratome, $NO_2$, Trialkylsilyl (3 bis 12 C-Atome), Hydroxy, Chlor, Brom, Jod oder Cyano,

$R^2$ für Wasserstoff, $-NH_2$, -CHO, Cyano, $-CH_2OH$ oder einen geradkettigen oder verzweigten, gesättigten oder ungesättigten Kohlenwasserstoffrest steht (1 bis 8 C-Atome)

und

$R^3$ für Wasserstoff oder einen geradkettigen oder verzweigten Alkylrest (1 bis 10 C-Atome) steht, der gegebenenfalls durch ein oder zwei Sauerstoffatome in der Alkylkette unterbrochen ist und der gegebenenfalls substituiert sein kann durch Fluor, Chlor, Brom, Jod, -CN, $-NH_2$, -OH oder Morpholino, in Form von Isomeren, Isomerengemischen, Racematen und optische Antipoden.

Von besonderem Interesse sind Verbindungen der allgemeinen Formel (I), in welcher

R für einen Phenyl-, Naphthyl-, Thienyl-, Pyrryl-, Imidazolyl-, Oxazolyl-, Isooxazolyl-, Thiazolyl-, Pyridyl-, Pyridazinyl-, Pyrimidyl-, Chinolyl-, Isochinolyl-, Indolyl-, Benzimidazolyl-, Chinazolyl-, Thionaphthenyl-, Chromonyl-, Thiochromonyl-, Chromenyl-, Thiochromenyl-, Benzoxadiazolyl- oder Benzthiadiazolylrest steht wobei die genannten

Reste gegebenenfalls 1 bis 3 gleiche oder verschiedene Substituenten aus der Gruppe Alkyl- (1 bis 15 Kohlenstoffatome), Alkenyl- (2 bis 15 Kohlenstoffatome), Alkinyl (2 bis 15 C-Atome), Alkoxy (1 bis 15 C-Atome), Fluor, Chlor, Brom, Jod, Trifluormethyl, Monofluoralkoxy- (1 bis 5 C-Atome), Polyfluoralkoxy- (1 bis 5 C-Atome), Hydroxy, Amino, Monoalkylamino- (1 bis 5 C-Atome), Dialkylamino- (1 bis 5 C-Atome), Nitro, Cyano, Azido, Carboxy, Carbonamido, Sulfonamido, $SO_m$-Alkyl (m = 0 bis 2, 1 bis 10 C-Atome), Phenyl-, Benzyl-, Benzyloxy- oder Benzylthio- enthalten können, wobei die 4 letztgenannten Substituenten gegebenenfalls 1 bis 3 Reste aus der Gruppe Alkyl (1 bis 4 C-Atome), Alkoxy (1 bis 4 C-Atome), Alkylthio (1 bis 4 C-Atome), Fluor, Chlor, Cyano, Nitro, Hydroxy, Trifluormethyl-, Amino-, Alkylamino- (1 bis 4 C-Atome) oder Dialkylamino- (1 bis 4 C-Atome) enthalten können,

$R^1$ einen geradkettigen, verzweigten oder cyclischen gesättigten oder ungesättigten Kohlenwasserstoffrest (1 bis 15 C-Atome) darstellt, der gegebenenfalls durch 1 oder 2 Sauerstoff- oder Schwefelatome in der Kette unterbrochen sein kann und der gegebenenfalls substituiert sein kann durch ein oder mehrere Fluoratome, $NO_2$, Trialkylsilyl (3 bis 9 C-Atome), Hydroxy, Chlor oder Cyano,

$R^2$ für Wasserstoff, -CHO, -Cyano oder einen geradkettigen oder verzweigten, gesättigten oder ungesättigten Kohlenwasserstoffrest steht (1 bis 6 C-Atome)

und

$R^3$ für Wasserstoff oder einen geradkettigen oder verzweigten Alkylrest (1 bis 6 C-Atome) steht, der gegebenenfalls durch ein oder zwei Sauerstoffatome in der Alkylkette unterbrochen ist und der gegebenenfalls substituiert sein kann durch Fluor, Chlor, Cyano, Hydroxy oder Morpholino.

Vorzugsweise seien solche Verbindungen der allgemeinen Formel (I) genannt, in welcher

R für einen Phenyl-, Naphthyl-, Pyridyl- oder Thiochromenylrest steht, wobei die genannten Reste gegebenenfalls 1 bis 2 gleiche oder verschiedene Substituenten aus der Gruppe Alkyl (1 bis 5 Kohlenstoffatome), Alkoxy (1 bis 5 C-Atome), Chlor, Trifluormethyl, Difluoralkoxy- (1 bis 5 C-Atome), Nitro, Cyano, Azido, $SO_m$-Alkyl (m = 0 bis 2, 1 bis 5 C-Atome), Phenyl- enthalten können, wobei der letztgenannte Phenylrest gegebenenfalls 1 bis 2 Reste aus der Gruppe Alkyl (1 bis 3 C-Atome), Alkoxy (1 bis 3 C-Atome), Fluor, Chlor, Trifluormethyl-, enthalten können,

$R^1$ für einen geradkettigen oder verzweigten Alkylrest (1 bis 5 C-Atome) steht, der gegebenenfalls

durch ein Sauerstoff- oder Schwefelatom in der Kette unterbrochen sein kann und der gegebenenfalls substituiert sein kann durch ein oder mehrere Fluoratome, oder Trialkylsilyl (3 bis 6 C-Atome),

$R^2$ für Wasserstoff oder einen geradkettigen oder verzweigten, Alkylrest steht (1 bis 4 C-Atome),

und

$R^3$ für Wasserstoff oder einen geradkettigen oder verzweigten Alkylrest (1 bis 4 C-Atome) steht, der gegebenenfalls durch ein Sauerstoffatom in der Alkylkette unterbrochen ist und der gegebenenfalls substituiert sein kann durch Morpholino.

Die erfindungsgemäßen Verbindungen der allgemeinen Formel (I) können hergestellt werden, indem man Benzylidenverbindungen der allgemeinen Formel (II)

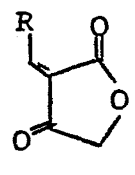

(II)

in welcher

R die oben angegebene Bedeutung hat

mit Enaminen der allgemeinen Formel (III)

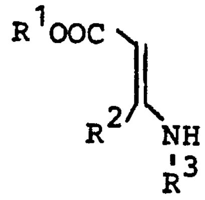

(III)

in welcher

$R^1$, $R^2$ und $R^3$ die oben angegebenen Bedeutungen haben,

gegebenenfalls in Gegenwart inerter organischer Lösungsmittel bei Temperaturen zwischen 0° und 150° umsetzt.

Die Mengenverhältnisse der Reaktanden zueinander sind beliebig, bevorzugt werden äquimolare Mengen eingesetzt, jedoch kann (III) in bis zu fünffachem Überschuß eingesetzt werden.

Die Umsetzungstemperatur beträgt vorzugsweise 20° bis 60°C, wenn in Gegenwart inerter Lösungsmittel und 40° bis 140°C, wenn ohne Lösungsmittel gearbeitet wird.

Als Lösungsmittel kommen alle inerten Lösungsmittel in Betracht, wie beispielsweise Alkohole, vorzugsweise tert.-Butanol oder Aromaten wie Toluol oder Benzol.

Die zur Herstellung verwendeten Benzylidenverbindungen (II) sind bekannt oder können nach bekannten Verfahren hergestellt werden (J. Org. Chem. 43, 1541 (1978) und Z. Chem. 10, 341 (1970)).

Die als Ausgangsstoffe verwendeten Aminocrotonsäureester (III) sind bekannt oder können nach bekannten Verfahren hergestellt werden (Cope, J. Amer. Chem. Soc. 67, 1017 (1945)).

Die erfindungsgemäßen Verbindungen (I) spalten in schwach saurer Lösung Wasser ab und gehen somit nach oraler Gabe in die 1,4-Dihydropyridinlactone (IV) über, die möglicherweise die eigentlich wirksamen Verbindungen darstellen.

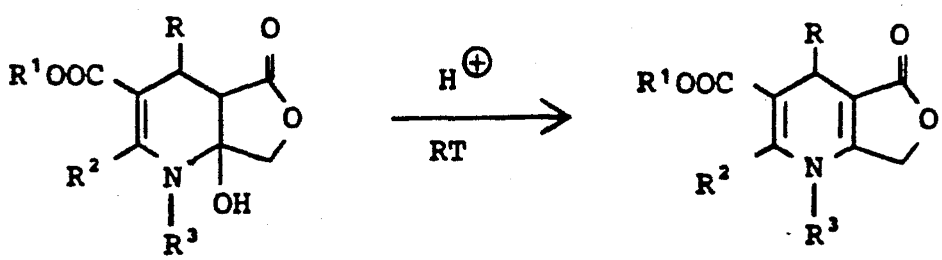


(I) (IV)

Als Lösungsmittel zur Überführung von (I) in (IV) kommen alle inerten Lösungsittel in Betracht, wie beispielsweise Alkohole, z.B. Ethanol, Aromaten wie Toluol oder Ester wie Ethylacetat.

Als Katalysator kommen alle Mineralsäuren in Betracht, wie z.B. Salzsäure oder Schwefelsäure.

Die Reaktionstemperatur beträgt -30° bis Siedetemperatur des Lösungsmittels, vorzugsweise 0° bis +50°C.

Die erfindungsgemäßen Verbindungen zeigen wie die bereits beschriebenen Verbindungen IV ein wertvolles

pharmakologisches Wirkungsspektrum. Sie können als Cardiotonika zur Verbesserung der Herzkontraktilität dienen. Darüber hinaus können sie dadurch, daß sie den $Ca^{++}$-Einstrom in die Zelle erhöhen, als Antihypotonica, zur Senkung von Blutzucker, zur Abschwellung von Schleimhäuten und zur Beeinflussung des Salz- und/oder Flüssigkeitshaushaltes eingesetzt werden.

Die erfindungsgemäßen Verbindungen können in bekannter Weise in die üblichen Formulierungen überführt werden, wie Tabletten, Kapseln, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß-/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum,Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt bevorzugt oral. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixiere können die Wirkstoffe außer den obengenannten Hilfsstoffen mit verschiedenen Geschmacksaufbesserern oder Farbstoffen versetzt werden.

Im allgemeinen hat es sich als vorteilhaft erwiesen, zur Erzielung wirksamer Ergebnisse eine Dosierung von

etwa 0,01 bis 20 mg/kg, vorzugsweise 0,1 bis 10 mg/kg Körpergewicht zu verabreichen.

Trotzdem kann es gegebenenfalls erforderlich sein, von den genannten Mengen abzuweichen, und zwar in Abhängigkeit vom Körpergewicht des Versuchstiers bzw. der Art des Applikationsweges, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Beispiel 1

7a-Hydroxy-2-methyl-4-(3-nitrophenyl)-5-oxo-1,4,4a,5,6,7a-hexahydrofuro/̄3,4-b̲7pyridin-3-carbonsäuremethylester.

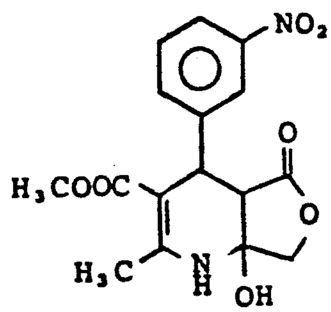

4 m Mol 3-(3-Nitrobenzyliden)-4-oxo-butyrolacton (E/Z-Gemisch) und 4,4 m Mol 3-Aminocrotonsäuremethylester werden in 5 g tert.-Butanol suspendiert und 3 h bei 30°C gerührt. Es wird bei Raumtemperatur einrotiert und der Rückstand mit wenig Methanol kristallisiert.

Fp: 147 - 149°C (aus Methanol):

Beispiel 2

7a-Hydroxy-2-methyl-4-(2-nitrophenyl)-5-oxo-1,4,4a,5,6-7a-hexahydrofuro/̄3,4-b̲7pyridin-3-carbonsäuremethylester

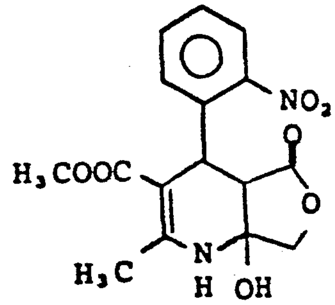

2 m Mol 3-(2-Nitrobenzyliden)-4-oxo-butyrolacton (E/Z-Gemisch) und 4 m Mol 3-Aminocrotonsäuremethylester werden gemischt und 1 h auf 90°C erwärmt.

Es wird mit Methanol versetzt und abgesaugt.

Fp: 163°C (Zers.).

Beispiel 3

7a-Hydroxy-2-methyl-4-(2-nitrophenyl)-5-oxo-1,4,4a,5,7,7a-hexahydrofuro/3,4-b/pyridin-3-carbonsäureethylester

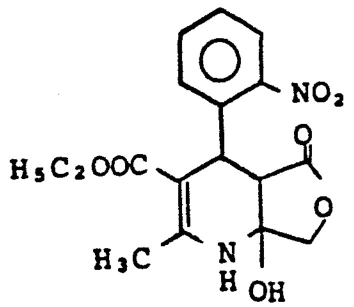

Darstellung analog Beispiel 2.

Fp: 160 - 161°C (Zers.).

Beispiel 4

7a-Hydroxy-2-methyl-4-(3-nitrophenyl)-5-oxo-1,4,4a,5,6,7a-hexahydrofuro/3,4-b/pyridin-3-carbonsäureethylester

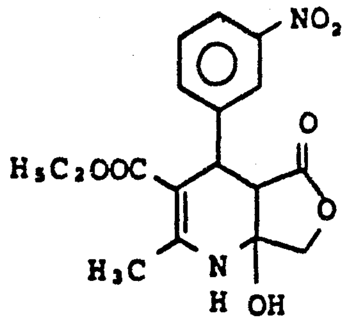

Darstellung analog Beispiel 1, jedoch 4 h Reaktionszeit.

Fp: 147°C.

Beispiel 5

7a-Hydroxy-2-methyl-4-(2-methylphenyl)-5-oxo-1,4,4a,5,7,7a-hexahydrofuro-/3,4-b/pyridin-3-carbonsäureethylester

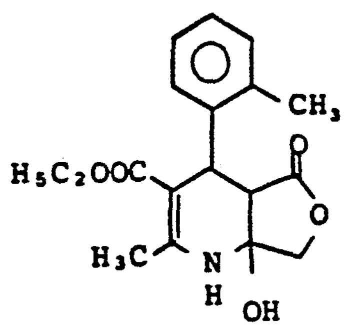

Darstellung analog Beispiel 1, Reaktionstemperatur: 30°C, Reaktionszeit: über Nacht.

Fp: 116 - 118°C.

Überführung in 1,4-Dihydropyridine

Beispiel 6

2-Methyl-4-(2-methylphenyl)-5-oxo-1,4,5,7-tetrahydrofuro-[3,4-b]pyridin-3-carbonsäureethylester

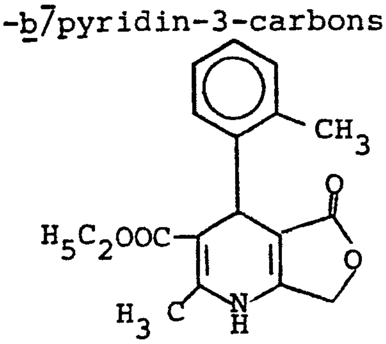

7a-Hydroxy-2-methyl-4-(2-methylphenyl)-5-oxo-1,4,4a,5,7,7a-hexahydrofuro-[3,4-b]pyridin-3-carbonsäureethylester wird in Ethanol gelöst und mit einem Tropfen ethanolischer HCl versetzt und anschließend eingeengt.

Fp: 196 - 198°C.

Beispiel 7

2-Methyl-4-(3-nitrophenyl)-5-oxo-1,4,5,7-tetrahydrofuro-[3,4-b]pyridin-3-carbonsäureethylester

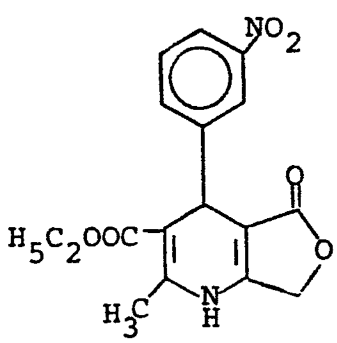

7a-Hydroxy-2-methyl-4-(3-nitrophenyl)-5-oxo-1,4,4a,5,7,7a-hexahydrofuro-[3,4-b]pyridin-3-carbonsäureethylester wird in Toluol gelöst, mit katalytischen Mengen p-Toluolsulfonsäure versetzt, kurz erwärmt, mit $Na_2CO_3$-Lösung ausgeschüttelt, getrocknet und eingeengt.

Fp: 229°C.

Patentansprüche

1. Hydroxytetrahydropyridinlactone der allgemeinen Formel I

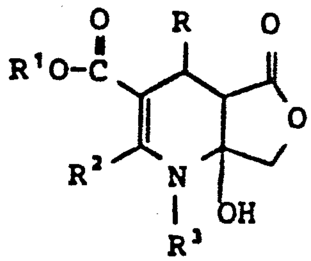

in der

R für einen Phenyl-, Naphthyl-, Thienyl-, Furyl-, Pyrryl-, Pyrazolyl-, Imidazolyl-, Oxazolyl-, Isooxazolyl-, Thiazolyl-, Pyridyl-, Pyridazinyl-, Pyrimidyl-, Pyrazinyl-, Chinolyl-, Isochinolyl-, Indolyl-, Benzimidazolyl-, Chinazolyl-, Chinoxalyl-, Thionaphthenyl-, Isothionaphthenyl-, Chromonyl-, Thiochromonyl-, Chromenyl-, Thiochromenyl-, Benzoxadiazolyl- oder Benzthiadiazolylrest steht, wobei die genannten Reste gegebenenfalls 1 bis 3 gleiche oder verschiedene Substituenten aus der Gruppe $C_1$-$C_{20}$-Alkyl, $C_2$-$C_{20}$-Alkenyl, $C_2$-$C_{20}$-Alkinyl, $C_1$-$C_{20}$-Alkoxy, Fluor, Chlor, Brom, Jod, Trifluormethyl, Monofluor-$C_1$-$C_{10}$-alkoxy-, Polyfluor-$C_1$-$C_{10}$-alkoxy-, Hydroxy, Amino, Mono-$C_1$-$C_{10}$-alkylamino-, Di-$C_1$-$C_{10}$-alkylamino-, Nitro, Cyano, Azido, Carboxy, Carb-$C_1$-$C_{10}$-

0144847

alkoxy, Carbonamido, Sulfonamido, $SO_m$-Alkyl (m = 0 bis 2, 1 bis 20 C-Atome), Phenyl-, Benzyl-, Benzyloxy- oder Benzylthio- enthalten können, wobei die 4 letztgenannten Substituenten gegebenenfalls 1 bis 3 Reste aus der Gruppe $C_1$-$C_5$-Alkyl, $C_1$-$C_5$-Alkoxy, $C_1$-$C_5$-Alkylthio, Fluor, Chlor, Brom, Jod, Cyano, Nitro, Azido, Hydroxy, Trifluormethyl-, Amino-, $C_1$-$C_6$-Alkylamino oder Di-$C_1$-$C_6$-alkylamino- enthalten können,

$R^1$ für einen geradkettigen, verzweigten oder cyclischen gesättigten oder ungesättigten Kohlenwasserstoffrest mit 1 - 20 C-Atomen steht, der gegebenenfalls durch 1 oder 2 Sauerstoff- oder Schwefelatome in der Kette unterbrochen sein kann und der gegebenenfalls substituiert sein kann durch ein oder mehrere Fluoratome, $NO_2$, $C_3$-$C_{12}$-Trialkylsilyl, Hydroxy, Chlor, Brom, Jod oder Cyano,

$R^2$ für Wasserstoff, $-NH_2$, -CHO, Cyano, $-CH_2OH$ oder einen geradkettigen oder verzweigten, gesättigten oder ungesättigten Kohlenwasserstoffrest mit 1 bis 8 C-Atomen steht,

und

$R^3$ für Wasserstoff oder einen geradkettigen oder verzweigten $C_1$-$C_{10}$-Alkylrest steht, der gegebenenfalls durch ein oder zwei Sauerstoffatome in der Alkylkette unterbrochen ist und der gegebenenfalls substituiert sein kann durch Fluor, Chlor, Brom, Jod, -CN, $-NH_2$, -OH oder Morpholino, in Form von Isomeren, Isomerengemischen, Racematen und optische Antipoden.

2. Verbindungen gemäß Anspruch 1, in welchen

R für einen Phenyl-, Naphthyl-, Thienyl-, Pyrryl-, Imidazolyl-, Oxazolyl-, Isooxazolyl-, Thiazolyl-, Pyridyl-, Pyridazinyl-, Pyrimidyl-, Chinolyl-, Isochinolyl-, Indolyl-, Benzimidazolyl-, Chinazolyl-, Thionaphthenyl-, Chromonyl-, Thiochromonyl-, Chromenyl-, Thiochromenyl-, Benzoxadiazolyl- oder Benzthiadiazolyl-rest steht, wobei die genannten Reste gegebenenfalls 1 bis 3 gleiche oder verschiedene Substituenten aus der Gruppe $C_1$-$C_{15}$-Alkyl-, $C_2$-$C_{15}$-Alkenyl-, $C_2$-$C_{15}$-Alkinyl-, $C_1$-$C_{15}$-Alkoxy-, Fluor, Chlor, Brom, Jod, Trifluormethyl, Monofluor-$C_1$-$C_5$-alkoxy-, Polyfluor-$C_1$-$C_5$-alkoxy-, Hydroxy, Amino, Mono-$C_1$-$C_5$-alkylamino-, Di-$C_1$-$C_5$-alkylamino-, Nitro, Cyano, Azido, Carboxy, Carbonamido, Sulfonamido, $SO_m$-Alkyl (m = 0 bis 2, 1 bis 10 C-Atome), Phenyl-, Benzyl-, Benzyloxy- oder Benzylthio- enthalten können, wobei die 4 letztgenannten Substituenten gegebenenfalls 1 bis 3 Reste aus der Gruppe $C_1$-$C_4$-Alkyl, $C_1$-$C_4$-Alkoxy, $C_1$-$C_4$-Alkylthio, Fluor, Chlor, Cyano, Nitro, Hydroxy, Trifluormethyl-, Amino-, $C_1$-$C_4$-Alkylamino- oder Di-$C_1$-$C_4$-alkylamino- enthalten können,

$R^1$ einen geradkettigen, verzweigten oder cyclischen gesättigten oder ungesättigten Kohlenwasserstoffrest mit 1 bis 15 C-Atomen darstellt, der gegebenenfalls durch 1 oder 2 Sauerstoff- oder Schwefelatome in der Kette unterbrochen sein kann und der gegebenenfalls substituiert sein kann durch ein oder mehrere Fluoratome, $NO_2$, $C_3$-$C_9$-Trialkylsilyl, Hydroxy, Chlor oder Cyano,

$R^2$ für Wasserstoff, -CHO, -Cyano oder einen geradkettigen oder verzweigten, gesättigten oder ungesättigten Kohlenwasserstoffrest mit 1 bis 6 C-Atomen steht,

und

$R^3$ für Wasserstoff oder einen geradkettigen oder verzweigten $C_1$-$C_6$-Alkylrest steht, der gegebenenfalls durch ein oder zwei Sauerstoffatome in der Alkylkette unterbrochen ist und der gegebenenfalls substituiert sein kann durch Fluor, Chlor, Cyano, Hydroxy oder Morpholino.

0144847

3. Verbindungen gemäß Anspruch 1, in denen

R für einen Phenyl-, Naphthyl-, Pyridyl-, Thiochromenylrest steht, wobei die genannten Reste gegebenenfalls 1 bis 2 gleiche oder verschiedene Substituenten aus der Gruppe $C_1$-$C_5$-Alkyl-, $C_1$-$C_5$-Alkoxy, Chlor, Trifluormethyl, Difluor-$C_1$-$C_5$-alkoxy-, Nitro, Cyano, Azido, $SO_m$-Alkyl (m = 0 bis 2, 1 bis 5 C-Atome), Phenyl-, enthalten können, wobei der letztgenannte Phenylrest gegebenenfalls 1 bis 2 Reste aus der Gruppe $C_1$-$C_3$-Alkyl, $C_1$-$C_3$-Alkoxy, Fluor, Chlor, Trifluormethyl-, enthalten können,

$R^1$ für einen geradkettigen oder verzweigten $C_1$-$C_5$-Alkylrest steht, der gegebenenfalls durch ein Sauerstoff- oder Schwefelatom in der Kette unterbrochen sein kann und der gegebenenfalls substituiert sein kann durch ein oder mehrere Fluoratome oder $C_3$-$C_6$-Trialkylsilyl,

$R^2$ für Wasserstoff oder einen geradkettigen oder verzweigten $C_1$-$C_4$-Alkylrest steht,

und

$R^3$ für Wasserstoff oder einen geradkettigen oder verzweigten $C_1$-$C_4$-Alkylrest steht, der gegebenenfalls durch ein Sauerstoffatom in der

Alkylkette unterbrochen ist und der gegebenenfalls substituiert sein kann durch Morpholino.

4. Verfahren zur Herstellung der Verbindungen gemäß Ansprüchen 1 - 4, dadurch gekennzeichnet, daß man Benzylidenverbindungen

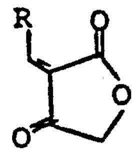

(II)

in welcher

R die in den Ansprüchen 1 - 3 angegebene Bedeutung hat

mit Enaminen der allgemeinen Formel (III)

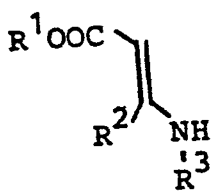

(III)

in welcher

$R^1$, $R^2$ und $R^3$ die in den Ansprüchen 1 - 3 angegebenen Bedeutungen haben

gegebenenfalls in Gegenwart inerter organischer Lösungsmittel bei Temperaturen zwischen 0° und 150°C umsetzt.

5. Verfahren gemäß Anspruch 4, dadurch gekennzeichnet, daß man die Verbindungen der Formeln II und III in äquimolaren Mengen einsetzt.

6. Verfahren gemäß Anspruch 4, dadurch gekennzeichnet, daß man die Verbindungen der Formel III im bis zu fünffachen Überschuß einsetzt.

7. Verfahren gemäß Ansprüchen 4 - 6, dadurch gekennzeichnet, daß man die Reaktion bei Temperaturen von 20 bis 60°C in Gegenwart inerter Lösungsmittel durchführt.

8. Verfahren gemäß Ansprüchen 4 - 6, dadurch gekennzeichnet, daß man die Reaktion bei Temperaturen von 40 bis 140°C ohne Lösungsmittel durchführt.

9. Verbindungen gemäß Ansprüchen 1 - 3 zur Bekämpfung von Erkrankungen.

10. Verwendung der Verbindungen gemäß Ansprüchen 1 - 3 zur Bekämpfung von Erkrankungen.

11. Verwendung der Verbindungen gemäß Ansprüchen 1 - 3 als Cardiotonika, als Antihypotonica, zur Senkung von Blutzucker, zur Abschwellung von Schleimhäuten und zur Beeinflussung des Salz- und/oder Flüssigkeitshaushaltes.

12. Arzneimittel enthaltend als Wirkstoff eine Verbindung gemäß Ansprüchen 1 - 3 neben üblichen Hilfs- und/oder Trägerstoffen.